Europäisches Patentamt

European Patent Office

Office européen des brevets

(11) Publication number: **0 174 367 B1**

# EUROPEAN PATENT SPECIFICATION

(45) Date of publication of patent specification: **10.06.92** (51) Int. Cl.⁵: **C12P 21/00**, C12N 1/20

(21) Application number: **85901721.2**

(22) Date of filing: **06.03.85**

(86) International application number:
**PCT/US85/00386**

(87) International publication number:
**WO 85/03949 (12.09.85 85/20)**

(54) **HOSTS AND METHODS FOR PRODUCING RECOMBINANT PRODUCTS IN HIGH YIELDS.**

(30) Priority: **06.03.84 US 586786**

(43) Date of publication of application:
**19.03.86 Bulletin 86/12**

(45) Publication of the grant of the patent:
**10.06.92 Bulletin 92/24**

(84) Designated Contracting States:
**AT BE CH DE FR GB LI LU NL SE**

(56) References cited:
| | |
|---|---|
| **EP-A- 0 055 945** | **EP-A- 0 088 540** |
| **EP-A- 0 123 228** | **EP-A- 0 128 733** |
| **EP-A- 0 147 178** | **WO-A-83/00702** |
| **WO-A-86/01538** | **GB-A- 2 119 804** |
| **US-A- 4 758 512** | |

**PROC. NATL. ACAD. SCI, vol. 80, March 1983, pages 1194-1198; R.A. YOUNG et al.: "Efficient isolation of genes by using anti-body probes"**

(73) Proprietor: **THE PRESIDENT AND FELLOWS OF HARVARD COLLEGE**
**17 Ouincy Street**
**Cambridge, MA 02138(US)**

(72) Inventor: **GOLDBERG, Alfred, L.**
**171 Crafts Road**
**Brookline, MA 02167(US)**
Inventor: **GOFF, Stephen A.**
**531-A Pleasant Street**
**Malden Massachusetts 02148(US)**
Inventor: **CASSON, Lawrence P.**
**46 Waldorf Road**
**New Highlands Massachusetts 02161(US)**

(74) Representative: **Bannerman, David Gardner et al**
**Withers & Rogers 4 Dyer's Buildings Holborn**
**London, EC1N 2JT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

PROC. NATL. ACAD. SCI, vol. 82, February 1985, pages 1074-1078; S. TABOR et al.: "A bacteriophage T7 RNA polymerase/promoter system for controlled exclusive expression of specific genes"

PROC. NATL. ACAD. SCI., vol. 81, November 1984, pages 6647-6651; S.A. GOFF et al.: "Heat shock regulatory gene htpR influences rates of protein degradation and expression of the lon gene in escherichia coli"

JOURNAL OF CELLULAR BIOCHEMISTRY SUPPLEMENT US, vol. 0, no. 9, part. B, page 237, abstract no. 1033, Geneva, CH; G.N. BUELL et al.: "Cloning of somatomedin-C double-stranded DNA"

FEDERATION PROCEEDINGS, vol. 42, no. 7, 1983, page 1832, abstract no. 434; G.T. MULLENBACH et al.: "The chemical synthesis, molecular cloning and expression in yeast of genes coding for human insulin-like growth factors I and II"

Bardwell and Craig, PNAS 81 : 848-852 (1984)

N. Roberts et al., Proc. Natl. Acad. Sci. USA, vol. 76, 1979, pp 760-764

N. Davies, Genetic Engineering 3, Williamson(ed.), ACademic Press, 1982, New York, pp 143-173

N. Shineberg et al., J. Bacteriol., vol. 116, 1973, pp 1469-1471

N. Yamamori et al., Heat Shock : From Bacteria To Man, Schlesinger et al(ed.), Cold Spring Harbor Laboratory,1982, Cold Spring Harbor, N.Y., pp 131-137

N. Neidhardt et al., Heat Shock:From Bacteria to Man, Schlesinger et al(ed.), Cold Spring Harbor Laboratory, 1982, Cold Spring Harbor, N.Y., pp 139-145

N. Philipps et al, J. Bacteriol., vol. 159, 1984, pp 283-287

N. Gottesman et al., J. BActeriol., vol. 133, 1978, pp 844-851

N. Neidhardt et al, Biochem. Biophys. REs. Comm., vol. 100, 1981, pp 894-900

N. Tobe et al., Mol. Gent. Genet., vol. 195, 1984, pp 10-16

N. Baker et al., Proc. Natl. Acad. Sci., USA, vol. 81, 1984, pp 6779-6783

N. Grossman et al, Cell, vol. 32, 1983, pp 151-159

N. Klapper et al, Chemical Abstracts, vol 99, 1983, Abstract no. 48021d, of Endiocrinology, vol. 112, 1983, pp 2215-2217

N. Houghton et al, Nucleic Acids REsearch, vol. 8, 1980, pp 2885-2894

## Description

The invention described herein was made in the course of work supported by a grant from the National Institutes of Health, U.S. Department of Health and Human Services.

## TECHNICAL FIELD OF INVENTION

This invention relates to improved host organisms and methods for producing recombinant products in high yields. More particularly, the present invention relates to host strains carrying specific mutations within their DNA sequences which cause the cells to exhibit a reduced capacity for degrading foreign products due to the reduced expression of cellular proteases and the use of these strains as host cells to produce increased yields of genetically engineered foreign proteins, polypeptides and other products. The methods disclosed in this invention advantageously permit the production, in high yields, of foreign recombinant proteins, polypeptides or other products in hosts which do not usually produce such products.

## BACKGROUND ART

The development of recombinant DNA technology has made possible the production of foreign products in host cells that have been transformed with foreign DNA sequences that code for those products. In general, the DNA coding for the desired amino acid, polypeptide or protein product is introduced into an appropriate site in an expression vehicle to form a recombinant DNA molecule. That molecule is then used to transform a compatible host and the host cultured to express the inserted DNA sequence and to produce the product coded for by that DNA sequence. Such recombinant techniques have been used for the production of eukaryotic and viral proteins in bacterial hosts. These include, for example, leukocyte interferon [S. Nagata et al., "Synthesis In E. coli Of A Polypeptide With Human Leukocyte Interferon Activity", Nature, 284, pp. 316-20 (1980)], antigens of human hepatitis B virus [C. J. Burrell et al., "Expression In Escherichia coli Of Hepatitis B Virus DNA Sequences Cloned In Plasmid pBR322", Nature, 279, pp. 43-47 (1979); M. Pasek et al., "Hepatitis B Virus Genes And Their Expression In E. coli", Nature, 282, pp. 575-79 (1979)], SV40t antigen [T. M. Roberts et al., "Synthesis Of Simian Virus 40t Antigen In Escherichia coli", Proc. Natl. Acad. Sci. USA, 76, pp. 5596-600 (1979)], and FMD viral antigens [H. Kupper et al., "Cloning Of cDNA Of Major Antigen Of Foot And Mouth Disease Virus And Expression In E. coli", Nature, 289, pp. 555-59 (1981)].

The large-scale commercial production of foreign proteins, polypeptides and other products by recombinant DNA techniques has often been limited due to intracellular degradation of the recombinant product by the host cell in which it is made. In a given host cell foreign or other abnormal proteins are rapidly degraded to peptides and amino acids. Furthermore, at high temperatures (above 40°C, for example), the degradation of these abnormal proteins, as well as normal cell proteins, has been found to be more rapid [S. Lin and I. Zabin, J. Biol. Chem., 247, pp. 2205-11 (1972); A. St. John et al., J. Biol. Chem., 253, pp. 3945-51 (1978)]. This is due to the fact that at such high temperatures, both normal and foreign proteins tend to be less structurally stable and become denatured, i.e., abnormal, with a resulting increase in their susceptibility to intracellular proteases.

This degradation of abnormal proteins requires metabolic energy [J. D. Kowit and A. L. Goldberg, "Intermediate Steps In The Degradation Of A Specific Abnormal Protein In Escherichia coli", J. Biol. Chem., 252, pp. 8350-57 (1977); K. Olden and A. L. Goldberg, "Studies Of The Energy Requirement For Intracellular Protein Degradation In Escherichia coli", Biochim. et Biophys. Acta., 542, pp. 385-98 (1978)]. An ATP-dependent protease, protease La, catalyzes the initial rate-limiting step in intracellular protein degradation [C. H. Chung and A. L. Goldberg, "The Product Of The lon (capR) Gene In Escherichia coli In The ATP-Dependent Protease, Protease La", Proc. Natl. Acad. Sci. USA, 78, pp. 4931-35 (1981); F. S. Larimore et al., "Studies Of The ATP-Dependent Proteolytic Enzyme Protease La In E. coli", J. Biol. Chem., 257, pp. 4187-95 (1982); L. Waxman and A. L. Goldberg, "Protease La From E. coli Hydrolyzes ATP And Proteins In A Linked Fashion", Proc. Natl. Acad. Sci. USA, 79, pp. 4883-87 (1982)]. Protease La is encoded by the lon gene, also referred to as the capR or deg gene [Chung and Goldberg, supra]. Mutants in the lon gene produce a defective but partially active protease [Chung and Goldberg, supra; C. H. Chung et al., "Studies Of The Protein Encoded By The lon Mutation capR9 In Escherichia coli: A Labile Form Of The ATP-Dependent Protease La That Inhibits The Wild-Type Protease", J. Biol Chem., 258, pp. 215-21 (1983)] and exhibit a reduced capacity to degrade abnormal proteins [S. Gottesman and D. Zipser, "deg Phenotype Of E. coli lon Mutants", J. Bacteriol., 133, pp. 844-51 (1978); Kowit and Goldberg, supra]. Although lon mutants exhibit a reduced capacity for protein degradation, they still show appreciable degradative activity

3

(approximately 1/3 to 1/2 normal activity). In addition, their use as hosts in recombinant DNA techniques is limited in terms of viability, due to several undesirable characteristic phenotypes of these lon mutants. These include greater sensitivity to ultraviolet radiation and overproduction of cell capsular mucopolysaccharides--mucoidy. Mutants which completely eliminate the function of the lon gene have not been previously available.

The potential of recombinant DNA technology in the commercially feasible production of useful amounts of foreign recombinant products has to date been limited by the absence of viable hosts characterized by a reduced capacity for degrading foreign products.

DISCLOSURE OF THE INVENTION

The present invention solves the problems referred to above by providing improved host organisms and methods for producing foreign proteins, polypeptides or other products in those host organisms transformed with DNA sequences coding for such products. More particularly, the present invention relates to host cell strains carrying specific mutations within their DNA sequences which cause the cells to exhibit a reduced capacity for degrading foreign products due to the reduced expression of cellular proteases and to the use of these strains to produce increased yields of genetically engineered foreign proteins, polypeptides and other products.

By virtue of this invention, it is possible to produce high yields of a desired foreign protein, polypeptide or other product in host cells which are characterized by a reduced capacity for degrading foreign products.

As will be appreciated from the disclosure to follow, the host strains and methods of this invention permit the large-scale production of foreign recombinant products in high yields.

BRIEF DESCRIPTION OF THE DRAWINGS

Figure 1 is a table which indicates the level of protease La isolated from htpR mutants at 30° and 42°C.

Figure 2 is a comparative graphical presentation of the percentage of degradation vs. time for various incomplete E.coli polypeptides that have incorporated puromycin.

Figure 3 is a comparative graphical presentation of the percentage of degradation vs. time for various abnormal E.coli proteins that contain canavanine in place of arginine.

BEST MODE OF CARRYING OUT THE INVENTION

In order that the invention herein described may be more fully understood, the following detailed description is set forth.

In the description, the following terms are employed:

Nucleotide - A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA Sequence - A linear array of nucleotides connected one to the other by phosphodiester bonds between the 3' and 5' carbons of adjacent pentoses.

Codon - A DNA sequence of three nucleotides (a triplet) which encodes, through its template or messenger RNA ("mRNA"), an amino acid, a translation start signal or a translation termination signal. For example, the nucleotide triplets TTA, TTG, CTT, CTC, CTA and CTG encode the amino acid leucine ("Leu"), TAG, TAA and TGA are translation stop signals and ATG is a translation start signal.

Polypeptide - A linear array of amino acids connected one to the other by peptide bonds between the $\alpha$-amino and carboxy groups of adjacent amino acids.

Gene - A DNA sequence which encodes through its mRNA a sequence of amino acids characteristic of a specific polypeptide.

Transcription - The process of producing mRNA from a gene or DNA sequence.

Translation - The process of producing a polypeptide from mRNA.

Expression - The process of producing a polypeptide from a DNA sequence or gene. It involves transcription and translation.

Plasmid - A nonchromosomal, double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet$^R$) transforms a cell previously sensitive to tetracycline into one which is resistant to it. A host cell transformed by a plasmid or vector is called a "transformant".

Phage or Bacteriophage - Bacterial virus many of which consist of DNA sequences encapsidated in a protein envelope or coat ("capsid").

Cloning Vehicle or Vector - A plasmid, phage DNA or other DNA sequence which is able to replicate in a host cell, characterized by one or a small number of endonuclease recognition or restriction sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance.

Cloning - The process of obtaining a population of organisms or DNA sequences derived from one such organism or sequence by asexual reproduction.

Recombinant DNA Molecule or Hybrid DNA - A molecule consisting of segments of DNA from different genomes (the entire DNA of a cell or virus) which have been joined end-to-end outside of living cells and have the capacity to infect some host cell and to be maintained therein.

Expression Control Sequence - A sequence of nucleotides that controls and regulates expression of genes or DNA sequences when operatively linked to those genes or DNA sequences. The term "operatively-linked" includes having an appropriate start signal in front of the gene or DNA sequence encoding the desired product and maintaining the correct reading frame to permit expression of the inserted DNA sequence under the control of the expression control sequence and production of the desired product encoded for by that gene or DNA sequence.

We have found that cells increase their content of proteolytic enzymes at high temperatures or in the presence of large amounts of foreign polypeptides. The present invention relates to host cell strains carrying specific mutations within their DNA sequences which cause the cells to exhibit a reduced capacity, inter alia, at high temperature or in the presence of large amounts of foreign proteins, for degrading foreign products. We believe that this important characteristic is due to the reduced expression of cellular proteases. Accordingly, the use of these host cells permits the production of increased yields of genetically engineered foreign proteins, polypeptides and other products.

In one preferred embodiment of this invention, the specific mutation or defect in the expression of protease genes is located in the htpR gene where it affects the production or function of that gene product. The htpR gene of E.coli is a regulatory gene involved in the heat-shock response of the cell. When "heat-shocked" by high temperature or other adverse conditions, e.g., the appearance of abnormal proteins due to the denaturation of some cellular proteins at high temperature, the cell responds by increasing the synthesis of a number of polypeptides referred to as "heat-shock proteins" [F. C. Neidhardt et al., "Molecular Cloning And Expression Of A Gene That Controls The High-Temperature Regulon Of Escherichia coli", J. Bacteriol., 153, pp. 597-603 (1983); T. Yamamori et al., "Escherichia coli Gene (hin) Control Of Transcription Of Heat Shock Operons And Cell Growth At High Temperatures"; F. C. Neidhardt, "The High Temperature Regulon Of Escherichia coli", in Heat Shock From Bacteria To Man, M. J. Ashburner et al. (eds.), Cold Spring Harbor Laboratory, pp. 131-38; 139-45 (1982)]. We have found that a signal for the heat-shock response is the appearance of those denatured proteins in the cell. We have also discovered that the accumulation of foreign polypeptides or other products in the cell as a result of cloning and expressing heterologous proteins or polypeptides also provokes that heat-shock response. Finally, we have determined that at least one of the heat-shock proteins plays a critical role in the proteolytic degradation of abnormal or foreign proteins [S. Goff et al., Proc. Natl. Acad. Sci. USA, 81, pp. 6647-51 (1984); T. A. Baker et al., Proc. Natl. Acad. Sci. USA, 81, pp. 6779-83 (1984)].

We have discovered that one of these heat-shock proteins is protease La, an ATP-dependent protease which carries out the rate-limiting step in the selective degradation of abnormal proteins within the cell [S. Goff et al., supra; T. A. Phillips et al., J. Bacteriol., 159, pp. 283-87 (1984)]. We have also discovered that protease La, as well as other heat-shock proteins, are induced even at low temperatures in response to the accumulation of foreign proteins within the cell. Our experimental evidence indicates that the production of large amounts of foreign proteins, as occurs on expression of foreign DNA sequences cloned in host cells transformed with those sequences, induces the production at low temperatures (e.g., 30°C) of several proteins that are also induced by heat-shock. For example, the expression of DNA sequences encoding tissue plasminogen activator (TPA) in host cells transformed with those sequences causes increased expression of the lon gene which encodes protease La even at low temperatures, such as 30°C. Thus, induction of the heat-shock proteins represents a generalized mechanism for the protection of the cell from the accumulation of foreign or other abnormal proteins at both high and low temperatures.

This induction of proteolytic enzymes in response to accumulated abnormal proteins is particularly problematic for the production of genetically engineered foreign proteins in high yields. We have found that upon production of a desired foreign protein in a host organism, protease La and other proteases are synthesized by the cell at increased rates and increase the cells capacity to degrade the desired protein. As a result, means were sought to overcome this obstacle to high yield production of genetically engineered proteins. We found that the induction of the heat-shock proteins does not occur in htpR mutants, also referred to as hin mutants. Such mutants can be obtained by those skilled in the art using standard mutagenesis techniques.

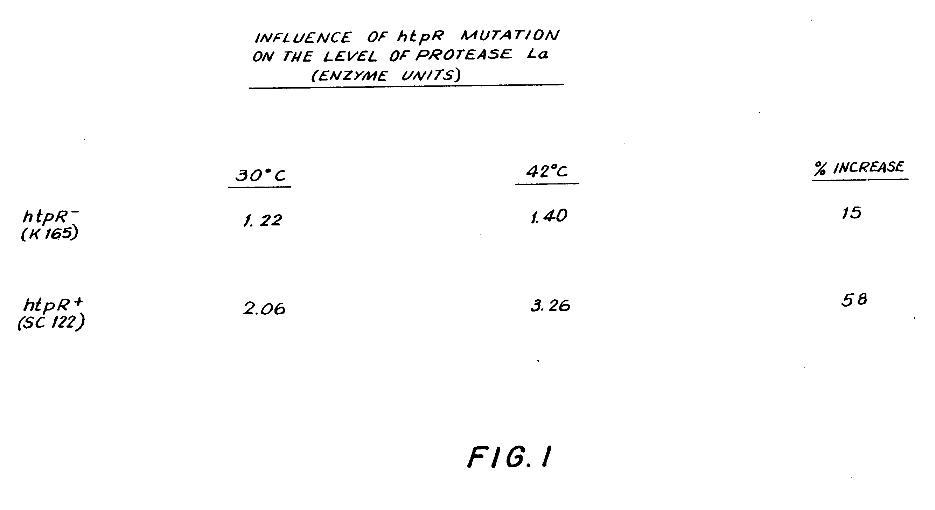

Our studies of the effect of htpR mutation on cellular protein degradation indicate that the htpR gene controls the transcription of the lon gene encoding protease La. In htpR mutants, there is reduced transcription of the lon gene, resulting in the reduced production of protease La which, in turn, leads to a decrease in the capacity of the mutant to degrade abnormal or foreign proteins. This reduced capacity to degrade aberrant proteins is evident under both high and low temperature conditions (e.g., 42°C and 30°C, respectively). Thus, the level of protease La isolated from mutant cells is lower at 30°C than in the wild type cell and does not increase on transfer to 42°C (Figure 1). The table in Figure 1 illustrates the level of protease La isolated from htpR mutants at 30° and 42°C. Data set forth in the table was obtained using E.coli cultures grown at 30° and shifted to 42°C for 1 hour. Protease La activity was measured with the specific fluorometric substrate glutaryl-alanyl-phenylalanyl-methoxynaphthylamine after partial purification of the enzyme by phosphocellulose chromatography, as described by L. Waxman and A. L. Goldberg in J. Biol. Chem., 260, pp. 12022-28 (1985). Furthermore, our studies showing increased expression of the lon gene after induction of genetically engineered TPA in host cells also demonstrated that this increased expression was absent in htpR mutants. The htpR mutations therefore appear to affect the basal level of protease La and prevent its increased production upon temperature shift.

The reduced degradative capacity exhibited by htpR mutants is greater than or equal to that found in lon mutants which encode a defective protease La. However, the htpR mutants do not demonstrate the undesirable phenotypes exhibited by lon mutants, such as the overproduction and accumulation of capsular polysaccharides (also referred to as mucoidy), defective cell division and filament formation and increased sensitivity to ultraviolet radiation or other DNA-damaging agents. The htpR mutants are therefore more viable than lon mutants under a variety of conditions encountered in large scale industrial fermentation (e.g., rich media). Whereas lon mutants grow poorly under these conditions, the htpR mutants growth well and therefore provide a distinct advantage as host organisms in recombinant DNA techniques to increase the yields of expressed foreign proteins, polypeptides, and other products.

Utilizing PI-mediated transduction, we have constructed htpR lon mutants which contain both an htpR mutation and a mutation within the lon gene, for example, lonΔ100 or capR9. These double mutants produce a labile protease in reduced amounts and exhibit less capability to degrade abnormal proteins than strains carrying either mutation alone. The defect in proteolysis by these double mutants is greater than that found in any known strain. These double mutants are viable hosts which achieve high cell densities in culture. Like the htpR mutants, they do not suffer from mucoidy or the other unhealthy characteristics exhibited by lon mutants.

Thus, the htpR lon mutants of this invention are particularly useful for the large-scale production of genetically engineered proteins which are usually subject to rapid intracellular degradation. According to this invention, a foreign protein, polypeptide or product may be produced using htpR mutants, and more preferably htpR lon mutants, by culturing a host transformed with a recombinant DNA molecule characterized by a DNA sequence coding for the foreign protein, polypeptide or product, said host being characterized by a defect in the expression of protease genes, and collecting the product from said culture. Foreign proteins, polypeptides and products may also be produced by culturing a host transformed with a recombinant DNA molecule characterized by a DNA sequence coding for the foreign protein, polypeptide or product, said host having a defect in the production or function of the htpR gene product.

The mutant strains of this invention may be advantageously utilized as host cells into which recombinant DNA sequences are introduced by any technique known in the art and expressed in the form of proteins, polypeptides and other products. The decreased capacity of these mutant strains to degrade foreign or other aberrant polypeptides permits the production of recombinant proteins and other products in high yields. For example, our experimental data indicate a three- to five-fold increase in the yield of genetically engineered tissue plasminogen activator produced in htpR lon mutants as compared to the yield obtained in wild type host cells.

The methods of this invention are applicable to the production of foreign proteins, polypeptides and products in prokaryotic and eukaryotic hosts including E.coli, bacilli, yeasts, fungi, animal or plant cells or other host organisms. The heat-shock response appears to be a universal mechanism for the protection of the cell from the adverse condition of high temperature [Ashburner et al., supra]. As suggested earlier [S. Goff et al., supra], the heat-shock response appears to be a general mechanism to protect the cell from the accumulation of abnormal protein molecules. We have observed ATP-dependent proteases analogous to protease La in other bacteria, such as Salmonella typhimeurium and Bacillus subtilis. Lon mutants have been detected in Salmonella typhimeurium. It is therefore likely that a mechanism for abnormal or foreign protein degradation similar to that found in E.coli is to be found in all prokaryotes. This mechanism may also exist in eukaryotic organisms. An enzyme similar to protease La has been found in the mitochondria of mammalian cells [M. Desautels and A. L. Goldberg, J. Biol. Chem., 257, p. 11673 (1982)] and in the cytosol of cultured mouse erythroleukemia cells [L. Waxman et al., J. Biol. Chem. 260, pages 11994 - 12000 (1985). The existence of such mechanisms in these other organisms permits the use of the methods of this invention to construct analagous mutant host organisms defective in the gene which functionally corresponds to the protease La gene present in E.coli and which, like the mutant organisms described herein, exhibit a reduced capacity to degrade foreign products.

In addition, any of a variety of expression vectors may be used to transform the host cells of the present invention. For example, useful expression vectors may consist of segments of chromosomal, non-chromosomal and synthetic DNA sequences, such as various known derivatives of SV40 and known bacterial plasmids, e.g., plasmids from E.coli including col E1, pCR1, pBR322, pMB9 and their derivatives, wider host range plasmids, e.g., RP4, phage DNAs, e.g., the numerous derivatives of phage λ, e.g., NM 989, and other DNA phages, e.g., M13 and Filamentous single-stranded DNA phages and vectors derived from combinations of plasmids and phage DNAs such as plasmids which have been modified to employ phage DNA or other expression control sequences or yeast plasmids such as the 2 μ plasmid or derivatives thereof. A particularly preferred expression vector is a high copy number plasmid or a bacteriophage derivative since these vectors provide an improved yield of expressed proteins.

Similarly, any of a variety of expression control sequences are available to improve the efficiency of expression (transcription and translation) of cloned DNA sequences. These expression control sequences are of two main types -- constitutive expression control sequences and controllable expression control sequences. Constitutive expression control sequences, such as β-lac, continuously function during host cell growth to express the cloned DNA sequence and to produce the product coded for by that DNA sequence. Controllable expression control sequences, such as trp, λP$_L$, or λP$_R$ may be regulated, i.e., switched on or off, during host cell growth so that expression of the cloned DNA sequence may be "switched on" at the most appropriate time in a culture's growth cycle. For example, controllable expression control sequences may be switched off to enable the host cells to propagate without excessive build-up of gene products and then switched on to promote the expression of large amounts of the desired protein products which are under the control of those expression control sequences. Because over-production of even normally non-toxic gene products may be harmful to host cells and lead to decreased stability of particular host-vector systems, a controllable expression control sequence is often favored so as to modulate expression during host cell growth.

Several controllable expression control sequences can be employed to express DNA sequences and genes coding for proteins and polypeptides in host organisms. These include, for example, the operator, promoter and ribosome binding and interaction sequences of the lactose operon of E.coli [e.g., K. Itakura et al., "Expression In Escherichia coli Of A Chemically Synthesized Gene For The Hormone Somatostatin", Science, 198, pp. 1056-63 (1977); D. V. Goeddel et al., "Expression In Escherichia coli Of Chemically Synthesized Genes For Human Insulin", Proc. Natl. Acid. Sci. USA, 76, pp. 106-10 (1979)], the corresponding sequences of the tryptophan synthetase system of E.coli [J. S. Emtage et al., "Influenza Antigenic Determinants Are Expressed From Haemagglutinin Genes Cloned In Escherichia coli", Nature, 283, pp. 171-74 (1980); J. A. Martial et al., "Human Growth Hormone: Complementary DNA Cloning And Expression In Bacteria", Science, 205, pp. 602-06 (1979)] and the major operator and promoter regions of phage λ [H. Bernard et al., "Construction Of Plasmid Cloning Vehicles That Promote Gene Expression From The Bacteriophage Lambda P$_L$ Promoter", Gene, 5, pp. 59-76 (1979)].

The fact that the htpR and htpR lon mutants of the present invention exhibit a reduced capacity to degrade foreign proteins at high temperatures makes them particularly suited for use in combination with temperature-inducible controllable expression control sequence. A heat-inducible expression sequence, the P$_L$ promoter of bacteriophage lambda,for example, can be fused to a DNA sequence encoding a desired protein by standard techniques known in the art. The resultant recombinant DNA sequence can then be used to transform an htpR or htpR lon mutant host organism. When grown at 42°C, such hosts express the desired protein or product to an enhanced degree, due to the temperature-induced expression control sequence switched on at that temperature. In addition, the expression of the desired protein or product in

7

further enhanced due to the reduced capacity of these hosts to degrade foreign proteins and products. In these htpR and htpR lon mutants, unlike wild type hosts, the increase in proteolytic enzymes at high temperature or upon production of foreign protein in the cells, does not occur.

Thus, use of the htpR and htpR lon mutants of this invention in combination with heat-inducible expression control sequences allows increased expression of genetically engineered proteins, polypeptides and other products. It is to be understood, however, that since the htpR and htpR lon mutants exhibit reduced degradation of foreign proteins at low temperatures (e.g., 30°C), the present invention also permits the enhanced expression of foreign proteins or other products utilizing non-temperature inducible as well as constitutive expression control sequences.

The host organisms and methods of this invention are applicable to the production of a wide variety of proteins and polypeptides. In addition, the methods of this invention may be employed to enhance the production of other products whose yields depend upon high levels of enzymes that are otherwise subject to degradation. For example, products such as the active components of vaccines or other pharmaceutically active products, agriculturally or other commercially useful compositions, enzymes, hormones, amino acids, industrial chemicals, antibiotics, foodstuffs, and the like can be usefully produced by the methods of this invention. Other products that may be produced by the methods of this invention include polypeptides displaying an immunological or biological activity of leukocyte interferon, fibroblast interferon or other interferons, polypeptides having the antigenicity of FMDV viral antigens or hepatitis B viral antigens, polypeptides displaying the biological activity of human or animal hormones such as somatomedin C and proinsulin and polypeptides and proteins displaying an immunological or biological activity of human, animal or viral products. The selection of a particular product is not part of this invention. Rather, the invention is applicable generally to the increased expression of DNA sequences coding for such products and to the production of such products in the mutant host organisms of the invention.

It should be noted that, although the present invention has been herein described as utilizing htpR and htpR lon mutants as host cells in recombinant DNA techniques, the htpR mutation itself may be inserted into host cells in which the corresponding gene has been eliminated. These host cells can then be transformed with recombinant DNA sequences, i.e., encoding the desired proteins, and will produce those proteins in high yields.

It should also be understood that the present invention also includes host organisms containing any of a number of possible mutations within the htpR or lon genes, or combinations thereof. Such mutants may be obtained utilizing standard mutagenesis and selection techniques to yield new strains defective in the heat-shock response such as those referred to by T. Tobe et al., "Isolation And Physical Mapping Of Temperature-Sensitive Mutants Defective In Heat-shock Induction Of Proteins In Escherichia coli, Mol. Gen. Genet., 195, pp. 10-16 (1984). Furthermore, the methods of this invention are applicable to the production of foreign proteins, polypeptides and products in strains of E.coli carrying other mutations within their DNA sequences which may cause the cells to exhibit a reduced capacity for degrading foreign proteins such as mutants which are defective in the induction of heat-shock proteins [see K. H. Paek, "A Defect In Induction Of Heat-Shock Proteins At High Temperature In xthA Mutants", in Abstracts Of Papers Presented At The Bacteriophage Meeting, August 21-26, p. 151, Cold Spring Harbor Laboratory, Cold Spring Harbor, New York (1984)].

In order that this invention may be better understood, the following examples are set forth. These examples are for purposes of illustration only and are not to be construed as limiting the scope of the invention in any manner.

Example 1

The following example is illustrative of the methods for constructing the htpR lon mutants of the present invention.

Construction Of htpR lon Mutants

E.coli cells (strain SG900, non-suppressor, lon$^+$, tsx$^+$, htpR$^+$) were infected with λNK55, a bacteriophage defective in DNA synthesis in non-suppressor hosts and which carries the Tn10 transposon in its CIII gene. The Tn10 transposon, a 9300 base pair transposable piece of DNA with inverted terminal repeat sequences, inserts itself into the tsx gene on the E.coli chromosome at approximately 10 min on the genetic map. The resulting region of the chromosome is designated tsx: Tn10.

Since Tn10 carries a gene for tetracycline resistance and since the transposon inserts itself into the tsx gene which codes for the bacteriophage T6 receptor, E.coli cells carrying the Tn10 insert will be

8

tetracycline resistant and resistant to T6 infection. The λND55 infected cells were, therefore, selected for resistance to tetracycline and these colonies further selected for resistance to T6 phage. Since the lon gene of E.coli is located near the tsx gene, the resulting isolates contained the lon gene linked to the tetracycline resistance marker of tsx: Tn10.

The linked lon and tsx: Tn10 genes were next introduced into a lon⁻ E.coli strain, such as lonΔ100, by PI-mediated transduction. Cells containing crossovers between the lon gene of the mutant strain and the tsx: Tn10 gene of the PI transducing element were selected for resistance to tetracycline and screened for the mucoid phenotype typical of lon mutants. The resulting isolates were lon mutants linked to the tetracycline resistance marker of Tn10 (lon⁻, tet$^r$).

To construct double mutant strains containing mutations in the lon gene and the htpR gene, which controls the heat shock response, the lon⁻ :tet$^r$ sequence of the above-described isolates was introduced into an htpR mutant E.coli strain, such as E.coli strain K165 or GW4701, lon$^+$, htpR⁻, by PI-mediated transduction. The double mutants were selected for tetracycline resistance and then screened for temperature sensitivity. It is to be understood that, although the double mutant described herein was constructed by introduction of the lon⁻ gene into an htpR⁻ strain, the reverse sequential construction, i.e., introduction of the htpR⁻ gene into a lon⁻ strain, may also be employed.

The resulting isolates, htpR⁻ lon⁻ double mutants, such as E.coli strain SG935 and SG927, lonΔ100, htpR⁻, may be used according to the present invention to increase the yields of genetically engineered foreign proteins produced in E.coli. Similarly, E.coli strains SG936 and SG928, lonR9, htpR⁻, may be produced and used according to the processes disclosed herein.

Example 2

Measurement Of Protein Degradation In htpR Mutants

The following example is illustrative of the methods for measuring protein degradation in the mutant strains of this invention.

A culture of the mutant strain to be examined was grown up overnight, preferably in minimal M9 media (J. H. Miller, Experiments In Molecular Genetics (Cold Spring Harbor Laboratory, 1981)). In the morning, 10 ml cultures were set up in Klett flasks and preferably the cells were allowed to grow at least two generations to verify that they were growing well.

When the cells were in early log phase, 0.2 ml-0.5 ml of 5 mg/ml $H_2O$ puromycin (filter sterilized) was added to each flask. The density of the cell cultures was such that at the end of the next 20 minute period, the cells would be in mid log phase. The final concentration of puromycin in each flask was 100-250 $\mu$g/ml. Puromycin is an inhibitor of protein synthesis which was incorporated into the growing polypeptides of the cells. This incorporation, after some time, resulted in the formation of abnormal prematurely terminated proteins. Such short proteins, like many cloned proteins of commercial interest, such as proinsulin, are ordinarily rapidly degraded by bacteria (A. L. Goldberg, Proc. Natl. Acad. Sci. USA, 69, pp. 2640-44 (1972)).

After addition of the puromycin, the cells were incubated for 15 min at 37°C. One $\mu$Ci of ³H-amino acid/ml was then added to the cultures to label the proteins being synthesized by the cells. (If the cells are grown in rich LB media, ³²S-methionine is the preferred label and 4 $\mu$Ci³⁵S-methionine/ml should be used.) The cultures were incubated with the radioactive label for 5 min at 37°C. The cells were immediately centrifuged in sterile 40 ml centrifuge tubes using a Sorvall SS-34 rotor for 1 min at 5000 rpm. The supernatant was rapidly decanted and the pellets washed by resuspension and vortexing in 2 volumes of media containing 1 mg/ml cold chaser amino acid. The cells were respun as before, resuspended in the cold chaser amino acid-containing media and transferred to Klett flasks. When transferred to these flasks, the cell density was adjusted such that at the end of the following 60 or 90 minute time course, the cells were not in stationary phase.

At this point in the procedure, the puromycin treated cells contained growing polypeptides with puromycin and labelled amino acids incorporated within their sequences. The puromycin caused these polypeptides to become disassociated from the ribosomes on which they were being synthesized and the resulting abnormal polypeptides were degraded by the bacterial cell.

To measure the rate at which a particular cell strain degraded these prematurely terminated proteins, samples were taken from the supernatant of the culture at different times and measured for free radioactive amino acids which resulted from the degradative process.

At t = 0, two 0.5 ml samples were taken from each culture. One was designated as "total counts" and represented total radioactive protein at t = 0. This sample was transferred to a tube maintained on ice and containing 100 $\mu$l of 70% TCA (trichloracetic acid). To this sample was added 0.1 ml of $H_2O$. The second

sample taken at t = 0 was designated "blank" and represented the acid soluble counts, i.e., free radioactive amino acids, present in the sample at t = 0. This 0.5 ml sample was also transferred to a tube on ice containing 100 $\mu$l of 70% TCA. The final TCA concentration was, preferably, 10%. To this sample was added 100 $\mu$l of 10% BSA (bovine serum albumin). At t = 15, 30, 60, and 90 minutes, similar 0.5 ml samples were taken from the cell culture and added to 10% TCA with 100 $\mu$l of 10% BSA.

Each sample was incubated on ice in the TCA solution for 30 min. Free radioactive amino acids in each sample were solubilized in the TCA, while nondegraded proteins remained insoluble and precipitated out of the TCA. Thus, protein degradation was determined by measuring the conversion of labelled proteins which were insoluble in TCA into free radioactive amino acids soluble in TCA. The production of TCA soluble counts over time was therefore utilized as an indication of the rate of protein degradation within the cells. It should be noted that the rate of degradation of the puromycyl proteins is very high and required performance of the above-described TCA precipitation steps as quickly as possible.

After incubation in TCA solution, all samples (except those for "total counts") were spun at 4°C in the IEC centrifuge, rotor #269, for 10 min at 3500 rpm and 0.4 ml of the supernatant of each sample was counted in 4 ml Liquiscint scintillation fluid for 5 min. The counts in each sample were used to calculate the % protein degradation based on the formula:

$$\% \text{ protein degradation} = \frac{\text{Average cpm (time point)-Blank cpm (t=0)}}{\text{Total counts cpm}} \times 100$$

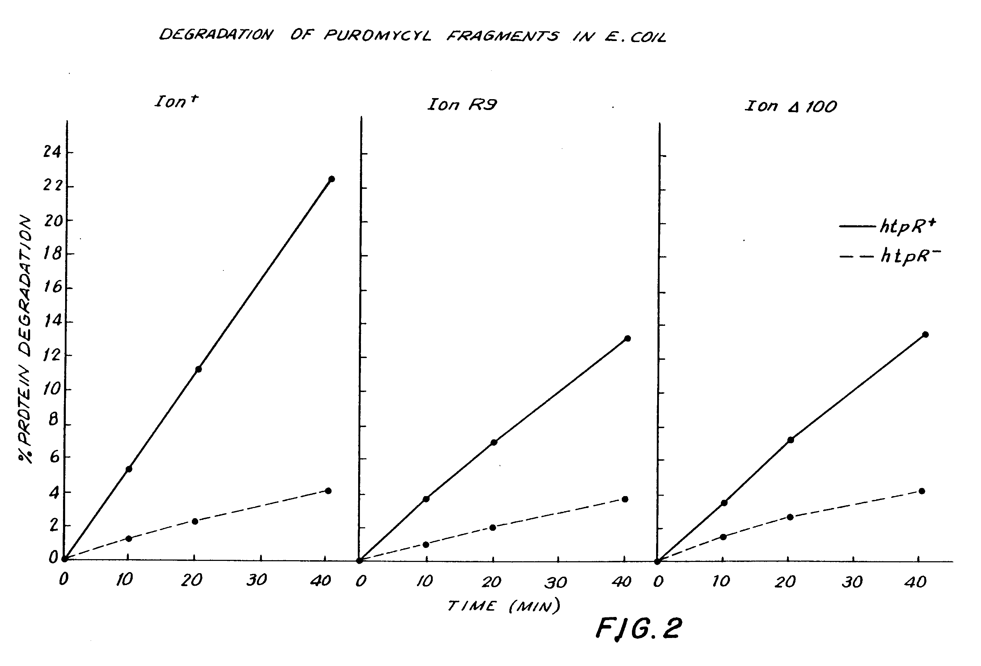

Figure 2 indicates the percentage of puromycyl protein degradation over time in a wild type E.coli cell (lon$^+$, htpR$^+$) versus an htpR mutant (lon$^+$, htpR$^-$) (see graph 1, using strain SC122 (htpR+) and K165 (htpR$^-$), gifts from Dr. Graham Walker) and the degradation in various lon mutants and htpR lon double mutants (see graphs 2 and 3). As is apparent from the figure, the htpR mutation significantly decreased the percentage of protein breakdown within the cell in both wild type and lon mutant cells.

While the above-described example involved degradation of short polypeptides resulting from the incorporation of puromycin, similar effects of the htpR mutation were also demonstrated by following the rapid degradation of full length abnormal proteins containing amino acid analogs such as canavanine. The procedure was similar to that described above. At mid-log phase, cells grown overnight in M9 media were centrifuged and resuspended in arginine-free media. Canavanine, an amino acid analog of arginine, was added to a 0.6mM final concentration. After 15 min, 10-20 $\mu$Ci $^3$H-phenylalanine was added. After 5 min the culture was centrifuged and resuspended in media containing 0.6mM arginine and cold phenylalanine at 0.5-1.0 mg/ml. The cultures were then respun and resuspended in the same media.

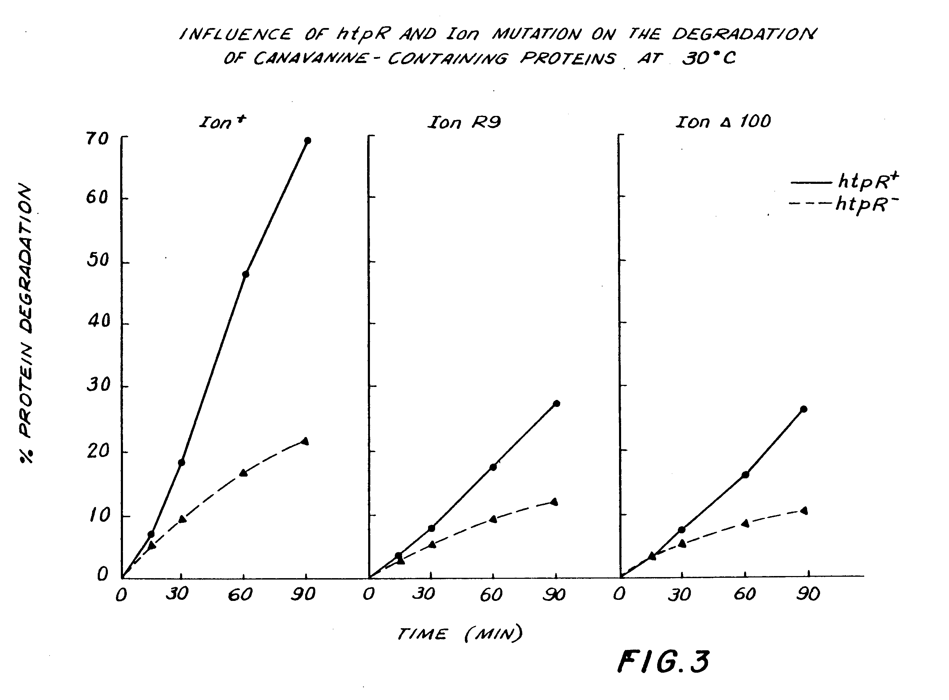

Samples were taken from the cultures in the identical manner as described above for the puromycin determination and the percentage of canavanine-containing proteins determined using the same formula. Figure 3 indicates the percentage of protein degradation over time of a wild type E.coli cell (lon$^+$, htpR$^+$) versus an htpR mutant (lon$^+$, htpR$^-$) (see graph 1, using strain GW1000 (htpR$^+$) and GW4701 (htpR$^-$), gifts from Dr. Graham Walker) and the protein degradation in various mutants and htpR lon double mutants (see graphs 2 and 3). The htpR mutation clearly decreased the protein breakdown in both the wild type and lon mutant cells. Furthermore, the double mutants, i.e., those carrying both htpR and lon mutations, showed a greater decrease in degradation than cells carrying only the htpR mutation alone, indicating that the double mutation provides some additive effect on protein degradation.

Example 3

Increased Expression of The lon Gene in htpR$^+$ Host Cells Producing Tissue Plasminogen Activator And Lack Of Such Expression In An htpR$^-$ Mutant

The following example demonstrates the increased expression of the lon gene, which codes for protease La, in host cells induced to produce a genetically engineered foreign protein, tissue plasminogen activator. E.coli host cells carrying an htpR mutation, however, did not exhibit this increased expression.

In order to easily detect turning on of the lon gene, a lon-lacZ operon fusion sequence was constructed which consisted of the lon promoter sequence operatively linked to the structural gene for $\beta$-galactosidase, lacZ. When the lon promoter is turned on, $\beta$-galactosidase is produced and can be easily assayed. This lon-lac fusion sequence was inserted into a λ bacteriophage and integrated into the host chromosome. A plasmid containing the TPA gene was then introduced into the same host, induced to produce TPA, and expression of the lon gene in the form of β-galactosidase activity was determined.

A. Construction Of A lon-lac Operon Fusion Sequence

A DNA sequence containing the lon promoter operatively linked to the lacZ gene was introduced into E.coli strains SC122 (F⁻ lac (am), trp (am), pho (am), mal (am), rpsL, supC (ts)) and its derivative, strain K165 (htpR (am)) as follows:

Plasmid pJMC40 containing the lon gene sequence (B. A. Zehnbauer et al., J. Bacteriol., 143, pp. 852-63 (1980) and an operon fusion vector, pMLB1022 (T. J. Silhavy et al., Experiments With Gene Fusions, Cold Spring Harbor Laboratories, Cold Spring Harbor, New York (1984)) were cut with EcoRI and BamHI restriction endonucleases (New England Biolabs) and the DNAs mixed together in the presence of T4 DNA ligase. pMLB1022 contains a gene for ampicillin resistance and a lacZ gene which encodes β-galactosidase, an enzyme involved in the fermentation of galactose, but the plasmid lacks the lac promoter for transcription of the structural lacZ gene.

E.coli cells that were lac⁻ were transformed with the ligation mixture and plated on MacConkey galactose plates containing 25 µg/ml ampicillin. Only those cells that were transformed with a plasmid containing the lon promoter of pJMC40 upstream from the lacZ gene of pMLB1022 will be able to produce β-galactosidase and will appear as red colonies on MacConkey galactose plates. Thus, ampicillin resistant colonies that were red on the plates were isolated. The plasmid DNA of one of these colonies was analyzed by restriction endonuclease mapping and agarose gel electrophoresis and found to contain the lon-lac operon fusion sequence. This plasmid was designated pSG1201.

We next integrated this fusion sequence into the chromosome of E.coli host cells by first inserting the fusion sequence into a λDNA from λNF1955 (I. Hui et al., J. Bacteriol., 152, pp. 1022-32 (1982)) which had also been restricted with EcoRI. The ligation reaction was carried out at a high concentration of λDNA to plasmid DNA utilizing 100 µg/ml of λNF1955 DNA and 3 µg/ml plasmid DNA. This high end to end concentration resulted in the formation of concatamers of recombinant λDNA in the ligation mixture. The ligation mixture was then packaged into λ particles using an in vitro packaging system (B. Hohn et al., Proc. Natl. Acad. Sci. USA, 71, pp. 2372-76 (1974).

λ-sensitive E.coli cells with lac deletions were infected with these recombinant phage particles and plated on TB (Bactotryptone) medium with 50 µg/ml X-Gal (5-bromo-4-chloro-3-indolyl-β-D-galactopyranoside), a substrate for β-galactosidase whose reaction with the enzyme causes the production of blue plaques. Blue plaques were isolated and the presence of the lon-lac operon fusion sequence verified by restriction analysis of the purified λDNA.

Since λNF1955 does not form stable lysogens and can only grow in suppressor strains, the recombinant phage particles were crossed with wild type λ phage, plated on non-suppressor cells in TB medium containing 50 µg/ml X-Gal at 37°C and screened for turbid blue plaques. A fusion sequence-containing phage capable of forming a stable lysogen was isolated in this matter and designated λ sigmagamma 101.

E.coli strains SC122 (htpR⁺) and K165 (htpR⁻) were transduced to mal⁺ in order to lysogenize the strains with λ sigmagamma 101 and were then plated on X-Gal-containing medium. Colonies which were blue and resistant to super-infection with λ were isolated.

Two strains isolated in this manner were designated E.coli SG943 (htpR⁺) derived from E.coli SC122 and E.coli SG942 (htpR⁻) derived from E.coli K165.

B. Expression of lon Genes In htpR⁺ v. htpR⁻ Cells Induced To Produce Tissue Plasminogen Activator

The lon-lac fusion-containing strains SG943 and SG942 were then transformed with plasmid pTPA102 which contained the DNA sequence encoding tissue plasminogen activator (an E.coli strain containing pTPA102 was deposited with the American Type Culture Collection, Rockeville, Maryland on August 27, 1984 under ATCC # 39810). pTPA102 was derived from plasmid pMC9 [R. A. Young et al., "Efficient Isolation Of Genes By Using Antibody Probes", Proc. Natl. Acad. Sci. USA, 80, pp. 1194-98 (1983)] by techniques well known in the art and contains the TPA gene under the control of the trc promoter (-35 from trp, -10 from lac). pTPA102 was used to transform E.coli strains SG943 and SG942, respectively, and each strain was grown up and split into two aliquots. To one aliquot of each strain, IPTG (isopropylthio-β-D-galactopyranoside), an inducer of the trc promoter, was added to a concentration of 1 mM. The other aliquot of each strain remained uninduced. The cells were incubated for 1 h at 30°C and then washed by filtration to remove the IPTG. The levels of β-galactosidase produced by each of the aliquots were determined by cleavage of ortho-nitrophenyl-$\beta$-D-galactopyranoside (Sigma) monitored at O.D.$_{240}$ [J. H. Miller, Experiments in Molecular Genetics, p. 431, Cold Spring Harbor Press (1972)]. Strain SG943 (htpR$^+$) showed an increase in $\beta$-galactosidase activity over time at 30°C whereas strain SG942 (htpR$^-$) showed no increase in $\beta$-galactosidase activity.

These results demonstrate that the lon promoter, which controls expression of protease La, is turned on by induction of TPA production in host cells transformed with the DNA sequence encoding the protein. htpR mutants, however, do not exhibit this increased expression.

Example 4

Induction Of Some Heat-Shock Proteins In Host Cells Producing Tissue Plasminogen Activator At 30°C And Lack Of This Induction In An htpR Mutant

pTPA102 containing the TPA gene was transformed into E.coli strains SG943 and SG942 as described above and the cells of each strain were divided into two aliquots. One aliquot of each strain was induced to produce TPA by the addition of IPTG for 1 h at 30°C as discussed above. The second aliquot was incubated for 1 h at 30°C without the addition of IPTG, i.e., was uninduced. The cells were then washed by filtration, resuspended in MOPS media containing $^{35}$S-methionine (>400 Ci/mmol, New England Nuclear) at 2 $\mu$Ci/ml and pulse-labeled for 5 min. Total cell protein was then extracted from the cells by TCA precipitation and washed first with 10% TCA, then 5% TCA and then with an ethanol/ether 1:1 solution. The protein was dried in vacuum, resuspended in 1x SDS sample buffer and run on a 7-15% linear gradient polyacrylamide gel. To demonstrate the classic heat-shock response, E.coli SG943 and SG942 cells that had not been transformed with pTPA102 were pulse labeled at 30°C and 42°C as described above. In the case of the cells grown at 42°C, the cells were shifted from 30°C to 42°C, allowed to grow for 5 min and then pulse-labeled.

A comparison of the autoradiographs of the untransformed htpR$^+$ cells grown at 30°C and 42°C showed the production at 42°C of numerous heat shock proteins (i.e., not present at 30°C). The autoradiograph of the TPA-containing htpR$^+$ cells that had been induced with IPTG at 30°C revealed 4-5 proteins which corresponded to some of the heat-shock proteins found in the autoradiograph of the htpR$^+$ cells that had been shifted to growth at 42°C (i.e., the classic heat-shock response). These 4-5 proteins were not present on the autoradiographs of the TPA-containing htpR$^-$ cells induced with IPTG nor were any heat-shock proteins present on the autoradiograph of the htpR$^-$ cells shifted to growth at 42°C. This data indicates that at least some of the proteins induced by heat-shock in htpR$^+$ cells are also induced by the production of TPA at 30°C whereas induction of these proteins is absent in htpR mutants.

Example 5

Increased Yields Of Tissue Plasminogen Activator Cloned Into htpR Mutants

Plasmid pTPA102 was transformed into a wild type E.coli strain, MC1061, a lon 100 mutant, SG2041, and an htpR lon double mutant, SG935, by techniques well known in the art. The cells were grown to an O.D. of 0.5 in LB medium and IPTG was added to a concentration of 1 mM for 0.5-1 h. The cells were spun down, resuspended in SDS sample buffer, boiled for 10 min and run on an SDS polyacrylamide gel. The gel was blotted onto nitrocellulose paper resulting in the transfer of the banded protein from the gel to the paper. The paper was then treated with rabbit antibody to TPA (a gift from Dr. Wolf-Dieter Schleuning) and then further treated with goat anti-rabbit antibody, this second antibody being conjugated with horseradish peroxidase (Biorad). The antibodies bound to those areas of the filter paper where TPA protein had banded. To detect the TPA band, the filter paper was exposed to $H_2O_2$ which caused a color change in the paper where the TPA protein had banded (see cite for Western blot). Using this procedure, we determined that the htpR lon double mutant exhibited a three- to five-fold increase in yield of tissue plasminogen activator over the wild type strain.

Example 6

Expression Of Somatomedin C In lon And htpR Mutants

The following example demonstrates the accumulation of the human hormone, somatomedin C (SMC) in lon, htpR and lon htpR mutant host cells following transformation of these mutants with a plasmid containing the gene encoding SMC and growth of the transformed host at 42°C. For example, we introduced plasmid pPLmuSMC$_{ori}$ or pPLmuSMC2 (which carry the gene for SMC), and pcl$_{857}$ into E.coli strains HB101, SG931 (wild type), SG933 (lon-), SG934(htpR$^-$) and SG936(lon$^-$htpR$^-$), respectively. We incubated these strains at 42°C for 2 h to induce the heat-shock response and detected the accumulation of SMC on Coomassie blue-stained SDS-polyacrylamide gels of E.coli extracts of the respective strains. Radioimmunoassay analysis of the five strains, each bearing pPLmuSMC$_{ori}$ and pcl$_{857}$, showed SMC ratios of 1:1:3:15:33 for the respective strains listed above. The lon htpR mutant therefore accumulated the most SMC and the two mutations were additive in their effects. Similar effects were seen in strains carrying pPLmuSMC2. In that case, the SMC ratios were 1:1:2.6:2.3:4.1 for the five respective strains. Again, the lon htpR mutant accumulated the most SMC and the two mutations exhibited additive effects.

We also performed a pulse-chase study detected by gel autoradiography. We grew cells overnight at 28°C in M9 minimal medium with all amino acids, except cysteine, followed by induction at 42°C in the same medium. The growing cultures were labelled for 30 seconds with [$^{35}$S]-cysteine and then chased by addition to the culture of an excess of unlabelled cysteine. HB101 bearing pPLmuSMC2 incorporated about four times more [$^{35}$S]-cysteine into SMC than HB101 bearing pPLmuSMC$_{ori}$. The half-life of SMC appeared to be 5 min or less in the wild-type HB101 host and SMC was almost undetectable after a 60 min chase. In contrast, the SMC in the lon htpR mutant host had a half-life of approximately 60 min.

Microorganisms prepared by the processes described herein are exemplified by cultures deposited in the American Type Culture Collection, Rockville, Maryland. Cultures deposited on March 5, 1984 are there identified as follows:

Culture SG935:     ATCC 39623
Culture SG936:     ATCC 39624

Cultures deposited on March 6, 1984 are there identified as follows:

Culture SG927:     ATCC 39627
Culture SG928:     ATCC 39628

While we have hereinbefore presented a number of embodiments of this invention, it is apparent that our basic construction may be altered to provide other embodiments which utilize the processes and compositions of this invention. Therefore, it will be appreciated that the scope of this invention is to be governed by the claims appended hereto rather than the specific embodiments which have been presented hereinbefore by way of example.

**Claims**

1. A method for producing a polypeptide in a bacterial host organism comprising the step of culturing a bacterial host transformed with a recombinant DNA molecule characterized by a non-htpR DNA sequence coding for said polypeptide, said host carrying a mutation in a heat-shock regulatory gene that results in a reduced proteolytic degrading capacity for said polypeptide.

2. A method for producing a polypeptide in a bacterial host organism comprising the steps of:
   a. inserting a non-htpR DNA sequence coding for a polypeptide into a cloning vehicle so that said DNA sequence is under the regulation of a functional expression control sequence;
   b. transforming a bacterial host with said cloning vehicle that has the inserted DNA sequence therein, said host carrying a mutation in a heat-shock regulatory gene that results in a reduced proteolytic degrading capacity for said polypeptide; and
   c. culturing said transformed host.

3. The method of claim 1 or 2 wherein the host organism is a strain of Bacillus.

4. The method of claim 1 or 2 wherein the host organism is a strain of Salmonella.

5. The method of claim 1 or 2 wherein the host organism is a strain of E.coli.

6. The method of claim 5 wherein said heat-shock regulatory gene is the htpR gene.

7. The method of claim 6 wherein the host is a lon mutant.

8. The method of claim 1 or 2 wherein the host is selected from the group consisting of E.coli strain SG 927, ATCC accession number 39,627; E.coli strain SG 928, ATCC accession number 39,628; E.coli strain SG 935, ATCC accession number 39,623; and E.coli strain SG 936, ATCC accession number 39,624.

9. The method of claim 1 or 2 wherein said DNA sequence is selected from the group consisting of DNA sequences that code for polypeptides displaying an immunological or biological activity of leukocyte interferon, fibroblast interferon, immune interferon, FMDV viral antigens, hepatitis B viral antigens, and proinsulin.

10. The method of claim 1 or 2 wherein said DNA sequence codes for a polypeptide displaying the biological activity of somatomedin C.

11. The method of claim 1 or 2 wherein said DNA sequence codes for a polypeptide displaying the biological activity of tissue plasminogen activator.

12. The method of claim 1 or 2 wherein the expression control sequence is a temperature-inducible expression control sequence.

13. A host selected from the group consisting of E.coli strain SG 927, ATCC accession number 39,627; E.coli strain SG 928, ATCC accession number 39,628; E.coli strain SG 935, ATCC accession number 39,623, and E.coli strain SG 936, ATCC accession number 39,624.

14. An E.coli host organism having mutations in both the htpR and lon genes which result in reduced proteolytic degrading capacity in response to the presence of a non-htpR protein, polypeptide or product.

15. A host according to claim 13 or 14 transformed with a recombinant DNA molecule comprising a DNA sequence coding for a non-htpR protein, polypeptide or product operatively linked to an expression control sequence in the recombinant DNA molecule.

**Revendications**

1. Procédé de production d'un polypeptide dans un organisme hôte bactérien qui comprend l'étape de mise en culture d'un hôte bactérien transformé par une molécule d'ADN recombinant caractérisé par une séquence d'ADN non htpR codant pour ledit polypeptide, cet hôte portant une mutation dans le gène de régulation du choc thermique ce qui a pour résultat une diminution de la capacité de dégradation protéolytique pour le polypeptide.

2. Procédé de production d'un polypeptide dans un organisme hôte bactérien qui comprend les étapes de :
   1. l'insertion d'une séquence d'ADN non htpR codant pour un polypeptide dans un véhicule de clonage de façon à ce que cette séquence d'ADN soit sous la contrôle d'une séquence de contrôle de l'expression,
   2. la transformation d'un hôte bactérien avec ce véhicule de clonage qui possède la séquence d'ADN insérée, ledit hôte portant une mutation dans le gène régulateur au choc thermique ce qui entraîne une diminution de la capacité de dégradation protéolytique pour ce polypeptide et
   3. une mise en culture dudit hôte transformé.

3. Procédé selon la revendication 1 ou 2, caractérisé en ce que l'organisme-hôte est une souche de Bacillus.

4. Procédé selon la revendication 1 ou 2, caractérisé en ce que l'organisme hôte est une souche de salmonella.

5. Procédé selon la revendication 1 ou 2, caractérisé en ce que l'organisme hôte est une souche d'E. Coli.

6. Procédé selon la revendication 5, caractérisé en ce que le gène régulateur du choc thermique est le gène htpR.

14

**7.** Procédé selon la revendication 6, caractérisé en ce que l'hôte est un mutant lon.

**8.** Procédé selon les revendications 1 ou 2, caractérisé en ce que l'hôte est choisi dans le groupe formé d'E. Coli souche SG927, ATCC numéro d'admission 39627) ; E. Coli souche SG928 ATCC numéro d'admission 39628 ; E. Coli souche SG935 ATCC numéro d'admission 39628 ; souche d'E. Coli souche SG935 ATCC numéro d'admission 39623 et E. Coli souche SG936, ATCC numéro 39624.

**9.** Procédé selon les revendications 1 ou 2, caractérisé en ce que ladite séquence d'ADN est choisie parmi le groupe consistant en les séquences d'ADN qui codent pour les polypeptides mettant en évidence une activité biologique ou immunologique d'interféron de leucocyte, d'interféron de fibroblaste, d'interféron immune, d'antigènes viraux FMDV, d'antigènes viraux de l'hépatite B et de la pro-insuline.

**10.** Procédé selon la revendication 1 ou 2, caractérisé en ce que cette séquence d'ADN code pour un polypeptide affichant l'activité biologique de la somatomédine C.

**11.** Procédé selon la revendication 1 ou 2, caractérisé en ce que cette séquence d'ADN code pour un polypeptide affichant l'activité biologique de l'activateur tissulaire de plasminogène.

**12.** Procédé selon la revendication 1 ou 2, caractérisé en ce que la séquence de commande de l'expression est une séquence de commande de l'expression thermo-inductible.

**13.** Hôte choisi dans le groupe, caractérisé en ce qu'il consiste en la souche d'E. Coli SG927 numéro d'admission ATCC 39627 ; la souche d'E. Coli SG928 numéro d'admission ATCC 39628 ; la souche d'E. Coli SG935 numéro d'admission ATCC 39623 ; et la souche d'E. Coli SG936 numéro d'admission 39624.

**14.** Organisme hôte d'E. Coli caractérisé en ce qu'il a des mutations à la fois dans les gènes htpR et lon qui ont pour conséquence une diminution de la capacité de dégradation protéolytique en réponse à la présence d'une protéine, d'un polypeptide ou d'un produit non-htpR.

**15.** Hôte selon la revendication 13 ou 14 transformé par une molécule d'ADN recombinant caractérisé en ce qu'il comprend une séquence d'ADN codant pour une protéine, un polypeptide ou un produit non htpR lié d'une manière fonctionnelle à une séquence de contrôle de l'expression dans la molécule d'ADN recombinant.

**Patentansprüche**

**1.** Verfahren zur Herstellung eines Polypeptides in einem bakteriellen Wirtsorganismus, umfassend den Schritt der Züchtung eines bakteriellen Wirtes, der mit einem rekombinanten, durch eine das Polypeptid codierende nicht-htpR DNA-Sequenz gekennzeichneten DNA-Molekül transformiert ist, wobei der Wirt eine Mutation in einem Hitzeschock-Regulatorgen trägt, die eine reduzierte proteolytische Abbaufähigkeit für das Polypeptid zur Folge hat.

**2.** Verfahren zur Herstellung eines Polypeptides in einem bakteriellen Wirtsorganismus, umfassend die Schritte

a. der Insertion einer das Polypeptid codierenden nicht-htpR DNA-Sequenz in ein Clonierungsvehikel, so daß die DNA-Sequenz von einer funktionalen Expressionskontrollsequenz reguliert wird;

b. der Transformation eines bakteriellen Wirtes mit dem Clonierungsvehikel, das die insertierte DNA-Sequenz enthält, wobei der Wirt eine Mutation in einem Hitzeschock-Regulatorgen trägt, die eine reduzierte proteolytische Abbaufähigkeit für das Polypeptid zur Folge hat; und

c. der Züchtung des transformierten Wirtes.

**3.** Verfahren nach Anspruch 1 oder 2, wobei der Wirtsorganismus ein Bacillus-Stamm ist.

**4.** Verfahren nach Anspruch 1 oder 2, wobei der Wirtsorganismus ein Salmonella-Stamm ist.

**5.** Verfahren nach Anspruch 1 oder 2, wobei der Wirtsorganismus ein E. coli-Stamm ist.

**6.** Verfahren nach Anspruch 5, wobei das Hitzeschock-Regulatorgen das htpR-Gen ist.

**7.** Verfahren nach Anspruch 6, wobei der Wirt eine lon-Mutante ist.

**8.** Verfahren nach Anspruch 1 oder 2, wobei der Wirt aus der Gruppe E.coli Stamm SG 927, ATCC Hinterlegungsnummer 39,627; E.coli Stamm SG 928 , ATCC Hinterlegungsnummer 39,628; E.coli Stamm SG 935, ATCC Hinterlegungsnummer 39,623 und E.coli Stamm SG 936, ATCC Hinterlegungsnummer 39,624 ausgewählt ist.

**9.** Verfahren nach Anspruch 1 oder 2, wobei die DNA-Sequenz aus der Gruppe von DNA-Sequenzen ausgewählt ist, die Polypeptide mit einer immunologischen oder biologischen Aktivität von Leukozyteninterferon, Fibroblasteninterferon, Immuninterferon, FMDV-Virusantigene, Heptatitis B-Virusantigene oder Proinsulin codieren.

**10.** Verfahren nach Anspruch 1 oder 2, wobei die DNA-Sequenz Polypeptide mit der biologischen Aktivität von Somatomedin C codiert.

**11.** Verfahren nach Anspruch 1 oder 2, wobei die DNA-Sequenz Polypeptide mit der biologischen Aktivität des Gewebe-Plasminogenaktivators codiert.

**12.** Verfahren nach Anspruch 1 oder 2, wobei die Expressionskontrollsequenz eine Temperatur-induzierbare Expressionskontrollsequenz ist.

**13.** Wirt, ausgewählt aus der Gruppe E.coli Stamm SG 927, ATCC Hinterlegungsnummer 39,627; E.coli Stamm SG 928, ATCC Hinterlegungsnummer 39,628; E.coli Stamm SG 935, ATCC Hinterlegungsnummer 39,623 und E.coli Stamm SG 936, ATCC Hinterlegungsnummer 39,624.

**14.** E.coli-Wirtsorganismus, der Mutationen sowohl im htpR-Gen als auch im lon-Gen aufweist, die eine reduzierte proteolytische Abbaufähigkeit aufgrund der Anwesenheit eines nicht-htpR-Proteins, -Polypeptids oder -Produktes zur Folge haben.

**15.** Wirt nach Anspruch 13 oder 14, transformiert mit einem rekombinanten DNA-Molekül, das eine ein nicht-htpR-Protein, -Polypeptid oder -Produkt codierende DNA-Sequenz umfaßt, die funktional mit der Expressionskontrollsequenz in dem rekombinanten DNA-Molekül verknüpft ist.

INFLUENCE OF htpR MUTATION
ON THE LEVEL OF PROTEASE La
(ENZYME UNITS)

| | 30°C | 42°C | % INCREASE |
|---|---|---|---|
| htpR⁻ (K 165) | 1.22 | 1.40 | 15 |
| htpR⁺ (SC 122) | 2.06 | 3.26 | 58 |

FIG. 1

EP 0 174 367 B1

DEGRADATION OF PUROMYCYL FRAGMENTS IN E.COIL

$Ion^+$    Ion R9    Ion Δ100

—— $htpR^+$
--- $htpR^-$

% PROTEIN DEGRADATION

TIME (MIN)

FIG.2

INFLUENCE OF htpR AND lon MUTATION ON THE DEGRADATION
OF CANAVANINE - CONTAINING PROTEINS AT 30°C

FIG.3